# EUROPEAN PATENT SPECIFICATION

(11) **EP 4 615 300 B1**
(45) Date of publication and mention of the grant of the patent: **08.07.2026**
(21) Application number: 23800896.5
(22) Date of filing: 18.10.2023
(51) Int. Cl.: A61B 1/00, A61B 1/24, A61C 9/00, A61B 1/32

(54) **DENTAL IMAGING DEVICES**
DENTALE BILDGEBUNGSVORRICHTUNGEN
DISPOSITIFS D'IMAGERIE DENTAIRE

(30) Priority: 10.11.2022 GB 202216752
(43) Date of publication of application: 17.09.2025
(73) Proprietor: ALSHAMSI, Mahra Mohamed, Al Khobar 34719, 2875 (SA)
(72) Inventor: RENFREW, Bruce, Leicester Leicestershire LE2 8PF (GB); WILLIAMSON, William, Leicester Leicestershire LE2 3PZ (GB); LANGFORD, Tobie, Leicester Leicestershire LE8 5PH (GB); ALSHAMSI, Mahra Mohamed, Al Khobar 34719, 2875 (SA)
(74) Representative: Serjeants LLP
(86) International application number: PCT/GB2023/052699
(87) International publication number: WO 2024/100372

(56) References cited:
- US-A1- 2013 209 954
- US-A1- 2020 138 553

## Description

### Technical Field

The present invention relates to dental imaging devices, and in particular to portable, hand-held, devices that may be used to capture a plurality of digital images for generating a three-dimensional image of the teeth and gums of a user. The devices may be used for open jaw and closed jaw imaging.

The present invention further relates to a system and method for generating a three-dimensional image of the teeth and gums of a user.

### Background Art

US 2013/209954 discloses a portable, hand-held, dental imaging device including a camera mount 220 with a flange 222 that is inserted into an opening 211 of a mouthpiece 210. This allows the camera mount 220 to be moved manually by a user relative to the mouthpiece 210. A mobile phone 280 with a digital camera is mounted to the camera mount 220. The digital camera is configured to capture a plurality of digital images of one of the upper and lower sets of teeth and gums of a user when the device is placed adjacent the mouth of the user. The digital camera is configured to be movable by the user along an arcuate track and to capture each digital image from a different position along the arcuate track. The device also includes a dental retractor 212a, 212b. The dental retractor 212a, 212b is insertable into the mouth of the user in use to retract the cheeks and expose the teeth and gums of the user and to position the device relative to and outside the mouth of the user when the plurality of digital images are being captured by the digital camera.

US 2020/138553 also discloses a dental imaging device, namely an intraoral scanner.

### Summary of the invention

The present invention provides a portable, hand-held, dental imaging device according to claim 1.

Each first digital image is captured by the first digital camera from a different position along the first arcuate (or curved) track. It will be readily understood that the first arcuate track is the path that the first digital camera follows, or along which it is moved, when the device is being used to capture the first digital images.

The first digital camera may be mounted on a first carrier. The first digital camera may be removably mounted on the first carrier, for example by inserting the first digital camera into a receiving part of the first carrier. The first carrier may be slidably engaged with a first guide (e.g., a fixed guide slot or guide rail) and may be moved along the first guide by the actuator, thereby moving the first digital camera along the first arcuate track. The first guide is arcuate or curved. The shape of the first guide will normally also determine the shape of the first arcuate track or path followed by the first digital camera.

Each second digital image is captured by the second digital camera from a different position along a second arcuate (or curved) track. The second digital camera is movable by the actuator or a separate actuator along the second arcuate track. It will be readily understood that the second arcuate track is the path that the second digital camera follows, or along which it is moved, when the device is being used to capture the second digital images.

The second digital camera may be mounted on a second carrier. The second digital camera may be removably mounted on the second carrier, for example by inserting the second digital camera into a receiving part of the second carrier. The second carrier may be slidably engaged with a second guide (e.g., a fixed guide slot or guide rail) and may be moved along the second guide by the actuator or the separate actuator, thereby moving the second digital camera along the second arcuate track. The second guide is arcuate or curved. The shape of the second guide will normally also determine the shape of the second arcuate track or path followed by the second digital camera.

For convenience, the plurality of first digital images captured by the first digital camera may be referred to below as a first set of digital images, and the plurality of second digital images captured by the second digital camera may be referred to as a second set of digital images.

The outer housing may be made of a suitable plastics material, for example. The housing may be substantially rigid and may enclose the various electronic and mechanical components of the device. The shape and configuration of the housing is preferably designed so that it is easy for the user to hold when the digital images are being captured, and will also be determined by the shape of the arcuate track or path followed by the digital cameras or the virtual line along which the digital cameras are arranged.

The first digital camera may be orientated downwards towards the lower set of teeth and gums of the user and the plurality of first digital images may be digital images of the lower set of teeth and gums of the user. The second digital camera may be orientated upwards towards the upper set of teeth and gums of the user and the plurality of second digital images may be digital images of the upper set of teeth and gums of the user.

Each digital camera may have any suitable construction and may be formed as a removable module, for example. Each digital camera may comprise a lens, an aperture, a digital image sensor and an image signal processor, for example. The lens of each digital camera may be configured to provide a suitable focal length/field of view for the required image capture. The image signal processor of each digital camera will typically process the data from the digital image sensor (i.e., from each photosite) to generate the digital images. It will be readily understood that the term "digital camera" will include any suitable imaging device or imaging sensor that is capable of capturing digital images.

The digital images may be stored preparatory to being transmitted to a separate image processor where they are processed to generate one or more three-dimensional images of the teeth and gums of the user.

The lens of each digital camera may be aligned with an imaging slot or opening in the housing. Each imaging slot or opening may be covered by a respective cover that is optically transparent or translucent. The lens of the first digital camera may be aligned with a first imaging slot in the housing as it moves along the first arcuate track and the lens of the second digital camera may be aligned with a second imaging slot in the housing as it moves along the second arcuate track. The first and second imaging slots in the housing may be arcuate or curved and may generally have the same shape as the respective first and second guides along which the first and second carriers are moved. The first imaging slot may be covered by a first cover that is optically transparent or translucent so that the first set of digital images may be captured through the first cover by the first digital camera. The second imaging slot may be covered by a second cover that is optically transparent or translucent so that the second set of digital images may be captured through the second cover by the second digital camera.

Because each digital image is captured at a different position (e.g., from a different point or location as the digital camera moves along its respective track), it means that each digital image will capture a slightly different view of the upper or lower teeth and gums of the user. **In** one arrangement, the first arcuate track or path followed by the first digital camera is preferably shaped so that the different views in the first set of digital images captured by the first digital camera are sufficient to allow for a good quality three-dimensional image of the one of the upper and lower sets of teeth and gums of the user to be generated. Similarly, the second arcuate track or path followed by the second digital camera is preferably shaped so that the different views in the second set of digital images captured by the second digital camera are sufficient to allow for a good quality three-dimensional image of the other one of the upper and lower sets of teeth and gums of the user to be generated. At the same time, the shape of each arcuate track or path must allow for a compact device that is easy for the user to hold and manipulate, and which may be a close fit against to the mouth of the user when the digital images are being captured. An arcuate or curved shape is generally preferred.

The actuator is an electric or mechanical actuator, for example the actuator may be an electric actuator such as an electric motor or stepper motor, or a mechanical actuator such as a Bowden mechanism. Both the first and second digital cameras may be moved by the same actuator or by separate actuators that may be combined or controlled in a co-ordinated way. Both digital cameras are preferably moved simultaneously along their respective track to minimise the imaging time. The first and second digital cameras may move in the same direction along their respective track when capturing digital images, or in opposite directions.

**In** one arrangement, digital images are captured by each digital camera at intervals as it moves along its respective track or path, e.g., as it moves from one end of the track to the other. Each digital camera may move along its respective track one or more times - for example, each digital camera may capture digital images as it moves from one end of the track to the other, and then back again. Any suitable number of digital images may be captured by each digital camera as it moves along its respective track. **In** other words, the first set of digital images may contain any suitable number of overlapping digital images captured by the first digital camera and the second set of digital images may contain any suitable number of overlapping digital images captured by the second digital camera. During a single use of the device, the number of digital images captured by each digital camera will normally be a trade-off between generating a good quality three-dimensional image and minimising the imaging time.

The device may further comprise a first lighting device that is preferably configured to illuminate the one of the upper and lower sets of teeth and gums of the user. The device may further comprise a second lighting device that is preferably configured to illuminate the other one of the upper and lower sets of teeth and gums of the user. The first lighting device may be positioned at a rear part of the device adjacent the first imaging slot (or the first cover) and the second lighting device may be positioned at the rear part of the device adjacent the second imaging slot (or the second cover).

Alternatively, the device may use a single lighting device to illuminate both the upper and lower sets of teeth and gums of the user.

Each lighting device may have any suitable shape and construction. For example, each lighting device may be a light emitting diode (LED) strip or similar. Each lighting device is preferably designed to provide consistent illumination of the teeth and gums of the user while the digital images are being captured. Consistent illumination is particularly important when considering that it will often be beneficial to compare three-dimensional images of the teeth and gums of the same user that are taken days or months apart. A comparison of two or more three-dimensional images for the same user taken at regular or irregular intervals may help to identify changes in their teeth or gums that may require dental treatment, such as the appearance of cavities, tooth decay, periodontitis, gingivitis, or the build-up of plaque, tartar etc. But it will also be understood that the need for dental treatment may be identified from a single three-dimensional image. Using one or more lighting devices to illuminate the teeth and gums of the user means that the three-dimensional images generated using the digital images captured by the device will be more consistent than simply relying on ambient lighting or an external light source, for example, which may be different each time the device is used. The three-dimensional images will also be generally consistent because the digital images used to generate the three-dimensional images are taken at the same position along the arcuate track by the same digital camera. Consistency between three-dimensional images captured at different times may also be helpful if the images are going to be processed by artificial intelligence, e.g., a machine-learning algorithm or similar software, which may be used to identify changes etc.

The device comprises a dental retractor that is preferably removably connected to the device. The dental retractor may be removably connected to the device by any suitable means, e.g., using a twist-fit or snap-fit connection. The dental retractor may have any suitable construction, and will generally be designed to retract the cheeks and expose the teeth and gums of the user so that the digital images may be captured. Because the connected dental retractor is part of the device, when it is inserted into the mouth of the user, it may ensure that the device is correctly positioned relative to the mouth - i.e., so that the teeth and gums are properly within the field of view of the digital cameras. The dental retractor may be re-useable or disposable. If the dental retractor is re-useable it may be made of a suitable material that is capable of sterilisation, for example. One device may be used by two or more users, with each user having their own dental retractor. Preparatory to using the device, the user may therefore connect their own dental retractor and then remove it afterwards. Different dental retractors may be used depending on whether the device is used for open jaw or closed jaw imaging, the age of the user etc.

The device may comprise a processor (or controller) and a memory device for storing the digital images after capture.

The device may comprise a communication device for transmitting the digital images, e.g., to an image processor. The communication device may use a suitable wireless protocol to transmit the digital images.

The device may comprise a power source, e.g., a rechargeable battery.

The device may comprise one or more control buttons, e.g., an on/off button and a button to start the capture of the digital images.

The device may comprise a speaker device. The speaker device may be used to provide audible instructions or warnings to the user when the device is being used.

The present invention further provides a system comprising the portable, hand-held, dental imaging device according to any of claims 1-6, and an image processor. The image processor is configured to process the plurality of first digital images captured by the dental imaging device to generate a three-dimensional image of the one of the upper and lower sets of teeth and gums of the user, e.g., using photogrammetry where three-dimensional coordinates may be derived from points within two-dimensional overlapping digital images taken from slightly different positions. The image processor may generate a three-dimensional image of the other one of the upper and lower sets of teeth and gums of the user if the device captures a plurality of second digital images.

The image processor may be a cloud-based processor or a software-based processor running on an electronic device to which the captured digital images are transmitted or uploaded. The image processor may execute one or more software programs or algorithms, e.g., photogrammetry software programs or algorithms, to generate the three-dimensional image.

The three-dimensional image generated by the image processor may be transmitted to an electronic device (including a mobile device such as a phone or tablet, for example) where it may be viewed by the user or a dental practitioner and/or stored. The three-dimensional image (or the captured digital images) may be processed by a machine-learning algorithm or similar software. Such processing may be carried out by a cloud-based processor or a software-based processor running on an electronic device to which the captured digital images or the three-dimensional image are transmitted or uploaded. The processing may also use biometric data of the user or which is relevant for a particular user.

The dental imaging device may be controlled remotely using an electronic device (including a mobile device such as a phone or table, for example) running a mobile application (or "app"). The app may also allow parameters relating to the digital image capture and subsequent processing to be customised for a particular user.

The present invention further provides a method of generating a three-dimensional image of the teeth and gums of a user using a portable, hand-held, dental imaging device according to any of claims 1-6, the method comprising:
inserting the dental retractor into the mouth of the user and placing the device adjacent and outside the mouth of the user,
using the first digital camera to capture a plurality of first digital images of one of the upper and lower sets of teeth and gums of the user, each first digital image being captured from a different position along the first arcuate track, and
processing the plurality of first digital images to generate a three-dimensional image of the one of the upper and lower sets of teeth and gums of the user.

Processing the plurality of first digital images to generate a three-dimensional image of one of the upper and lower sets of teeth and gums of the user may use photogrammetry, for example.

The device may be used to illuminate the one of the upper and lower sets of teeth and gums of the user while the first digital images are being captured.

The device may further comprise:
using the second digital camera to capture a plurality of second digital images of the other one of the upper and lower sets of teeth and gums of the user, each second digital image being captured from a different position along the second arcuate track, and
processing the plurality of second digital images to generate a three-dimensional image of the other one of the upper and lower sets of teeth and gums of the user. The processing may use photogrammetry, for example.

The device may be used illuminate the other one of the upper and lower sets of teeth and gums of the user while the second digital images are being captured.

The digital images may be transmitted from the dental imaging device to an image processor, for example using a suitable wireless protocol.

### Drawings

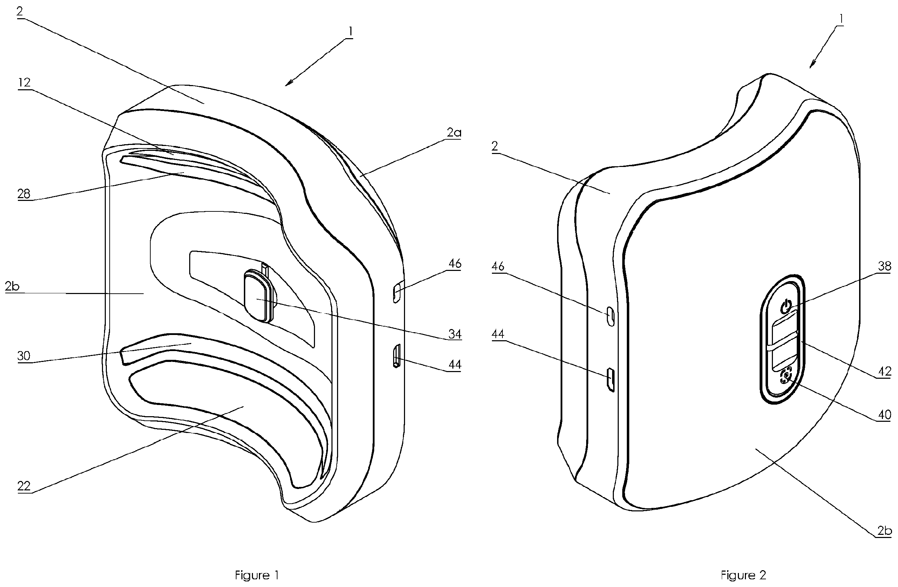

Figure 1 is a rear perspective view of a portable, hand-held, dental imaging device according to the present invention;
Figure 2 is a front perspective view of the dental imaging device of Figure 1;

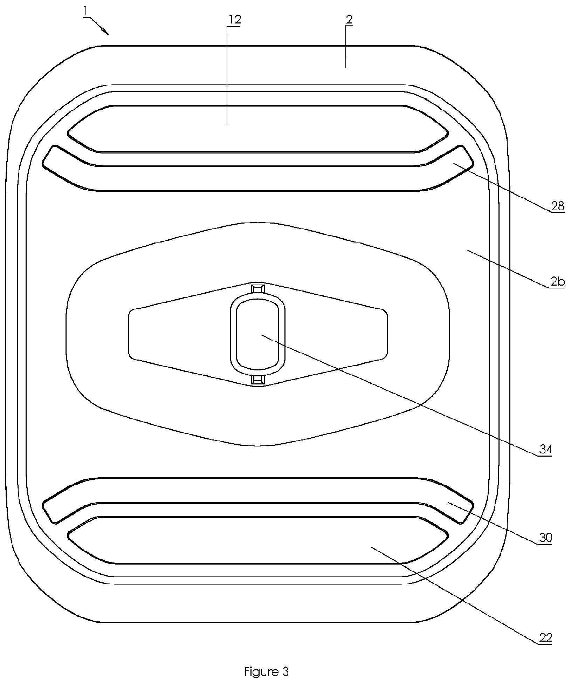

Figure 3 is a rear view of the dental imaging device of Figure 1;

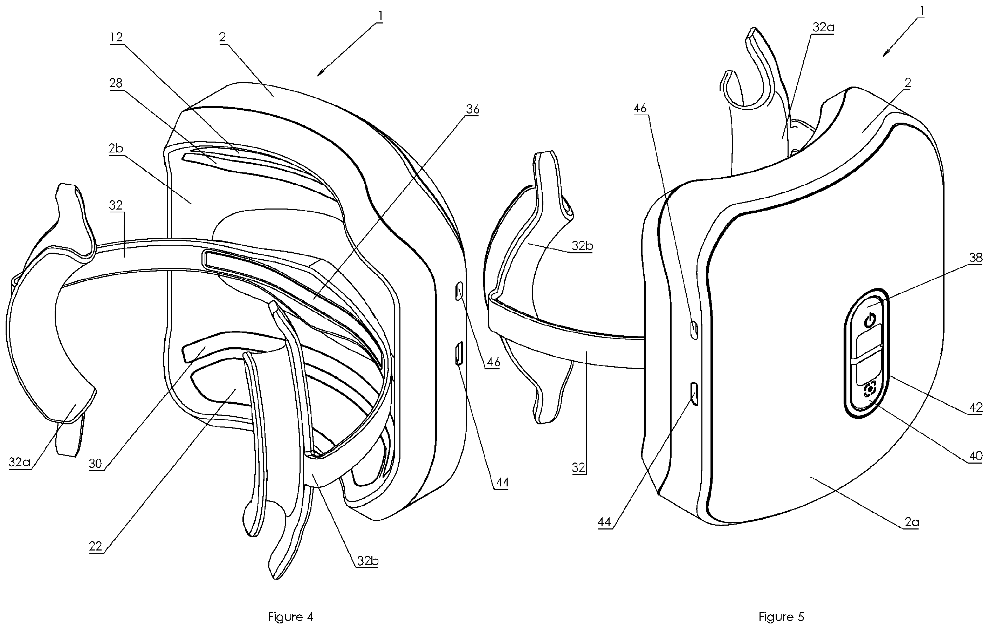

Figure 4 is a rear perspective view of the dental imaging device of Figure 1 with a dental retractor fitted;
Figure 5 is a front perspective view of the dental imaging device of Figure 4;
Figure 6 is a rear perspective view of the dental imaging device of Figure 1 with the rear part of the outer housing removed;
Figure 7 is a rear view of the dental image device of Figure 6; and
Figure 8 is a schematic view of a system according to the present invention.

Referring to the drawings, a portable, hand-held, dental imaging device 1 comprises a substantially rigid outer housing 2 made of a suitable plastics material.

The housing 2 has a front part 2a that is generally concave and a rear part 2b that is generally convex and which faces towards the mouth of the user when the device is used.

A first carrier 4 is positioned at an upper part of the housing 2 and is slidably engaged with an arcuate first guide slot 6. The first carrier 4 includes a receiving part 8 that is adapted to receive a first digital camera module (not shown). The first carrier 4 is moved backwards and forwards along the first guide slot 6 as described in more detail below. When the first digital camera module is mounted to the first carrier 4 it is aligned with an arcuate first imaging slot 10 at the upper part of the housing 2. The first imaging slot 10 is covered by an optically transparent or translucent first cover 12. The first cover 12 is formed on an upper sloping surface of the rear part 2b of the housing 2.

A second carrier 14 is positioned at a lower part of the housing 2 and is slidably engaged with an arcuate second guide slot 16. The second carrier 14 includes a receiving part 18 that is adapted to receive a second digital camera module (not shown). The second carrier 14 is moved backwards and forwards along the second guide slot 16 as described in more detail below. When the second digital camera module is mounted to the second carrier 14 it is aligned with an arcuate second imaging slot 20 at the lower part of the housing 2. The second imaging slot 20 is covered by an optically transparent or translucent second cover 22. The second cover 22 is formed on a lower sloping surface of the rear part 2b of the housing 2.

The field of view of the first digital camera is orientated downwards towards the lower set of teeth and gums of the user and the field of view of the second digital camera is orientated upwards towards the upper set of teeth and gums of the user.

The first and second carriers 4, 14 are moved backwards and forwards along their respective arcuate guide slots 6, 16 by a Bowden mechanism. It will be readily understood that when the first carrier 4 is moved along the arcuate first guide slot 6, the first digital camera will be moved along a corresponding arcuate track or path during which a plurality of first digital images are captured at different points along the track or path. When the second carrier 14 is moved along the arcuate second guide slot 16, the second digital camera will be moved along a corresponding arcuate track or path during which a plurality of second digital images are captured at different points along the track or path.

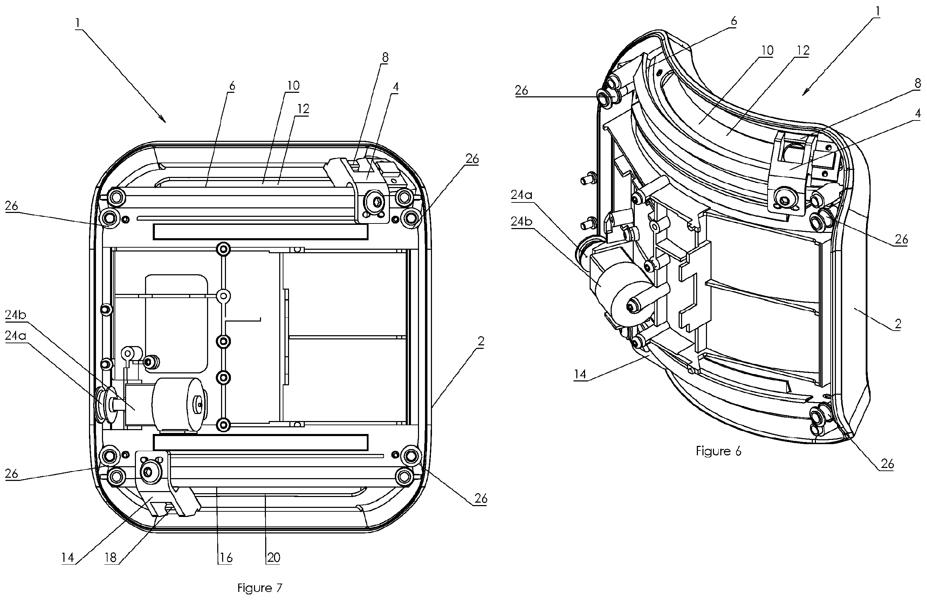

The Bowden mechanism includes a drive wheel 24a that is driven to rotate by a stepper motor 24b. A taut steel cable (not shown) extends around the drive wheel 24a and four bushes 26 to form a complete circuit and is fixedly connected to the first and second carriers 4, 14. Rotating the drive wheel 24a in opposite directions moves the cable around the bushings 26 in opposite directions, which in turn moves the first and second carriers 4, 14 backwards and forwards along their respective guide slots 6, 16. For example, if the drive wheel 24a is driven to rotate in a first direction (e.g., a clockwise direction), the first carrier 4 is moved along the first guide slot 6 by the cable in a first direction (e.g., from right to left as shown in Figure 7) and the second carrier 14 is simultaneously moved along the second guide slot by the cable in a second direction (e.g., from left to right as shown in Figure 7). A Bowden mechanism has the benefits that it can be driven by a single stepper motor and the cable allows for a tight bend radius, conforms well to the overall arcuate shape of the housing, and provides a highly reliable and robust connection to the first and second carriers. However, it will be readily understood that one or more different actuators may also be used to move the first and second carriers.

A first LED lighting strip 28 is positioned on the upper sloping surface next to the first cover 12 and orientated downwards to illuminate the lower set of teeth and gums of the user. A second LED lighting strip 30 is positioned on the lower sloping surface next to the second cover 22 and orientated upwards to illuminate the upper set of teeth and gums of the user.

A dental retractor 32 is removably connected to the device 1. In particular, the device 1 includes a protrusion 34 on the rear part 2b of the housing 2 as a mounting feature that may be received in a complementary opening or recess in the dental retractor 32. A base 36 of the dental retractor 32 is fitted on to the protrusion 34 and the dental retractor is then twisted to secure it to the device 1. For example, the dental retractor 32 may be fitted on the protrusion 34 and then twisted by about 90 degrees to fixedly secure it to the device 1. The dental retractor 32 may be removed from the device by twisting it by about 90 degrees in the opposite direction. Other types of fitting may also be used to releasably connect the dental retractor to the device, such as a snap-fit fitting, for example. The dental retractor 32 includes a pair of arms 32a, 32b that extend outwardly from the base part 36 and which are designed to retract the cheeks of the user and expose the teeth and gums so that the digital images may be captured by the device. The dental retractor 32 also positions the device 1 correctly with respect to the mouth of the user. A different dental retractor may be used depending on whether the device is being used for open jaw or closed jaw imaging, for example.

The device 1 has an on/off button 38 and an image capture button 40 on the front part 2a of the housing 2. The buttons are surrounded by a halo light 42.

The device 1 has a processor (not shown) and a memory device (not shown) for storing the digital images after capture. The device 1 also has a wireless communication device (not shown) for transmitting the digital images. The wireless communication device may be a wireless transmitter. When the device 1 is turned on, it may search for a suitable wireless connection (e.g., with a wireless router) and the halo light 42 will flash and then turn solid if a connection is established. If the wireless transmitter is using a Wi-Fi protocol, the WPS (or Wi-Fi Protected Setup) button on the wireless router may be pressed preparatory to establishing a wireless connection.

The device 1 has a battery (not shown) that may be charged through a charging port 44. The device may also be charged using a docking station, which may use wireless charging.

The device 1 includes a speaker device and port 46 through which audible notifications and instructions are provided to the user when the device is being used.

To use the device 1, it is turned on by pressing the on/off button 38. During a turn-on procedure, the device will establish a wireless connection. When the device 1 is ready for use, an audible notification will be provided to the user - i.e., that the device may be used for imaging.

If not done already, the user will connect the dental retractor 32 to the device 1.

The dental retractor 32 is inserted into the mouth of the user. The arms 32a, 32b of the dental retractor 32 may be gently squeezed together to insert them. When pressure is released, the arms 32a, 32b will retract the cheeks of the user and expose the teeth and gums of the user. The device 1 will be positioned at the correct distance from the mouth of the user and is supported by the hand of the user during the image capturing process.

When the user is ready for the device 1 to start capturing the digital images, the user presses the image capture button 40. The halo light 42 turns off and the first and second LED lighting strips 28, 30 turn on to illuminate the teeth and gums of the user.

The Bowden mechanism is controlled by the processor to move the first and second carriers 4, 14 so that the first and second digital cameras (not shown) are moved along their respective tracks or paths and are controlled to take a plurality of digital images at different points. For example, the first digital camera may take a total of seven digital images of the lower set of teeth and gums of the user at different points along the first track or path, and the second digital camera may take a total of seven digital images of the upper set of teeth and gums of the user at different points along the second track or path. It may take about 15 seconds for the first and second digital cameras to move from one end of the respective track or path to the other and for the digital images to be captured and temporarily stored.

Once the digital images have been captured, the halo light 42 turns on and the first and second LED lighting strips 28, 30 turn off. An audible notification is provided to the user - e.g., that the imaging is complete. The dental retractor 32 may be removed from the mouth and then removed from the device 1.

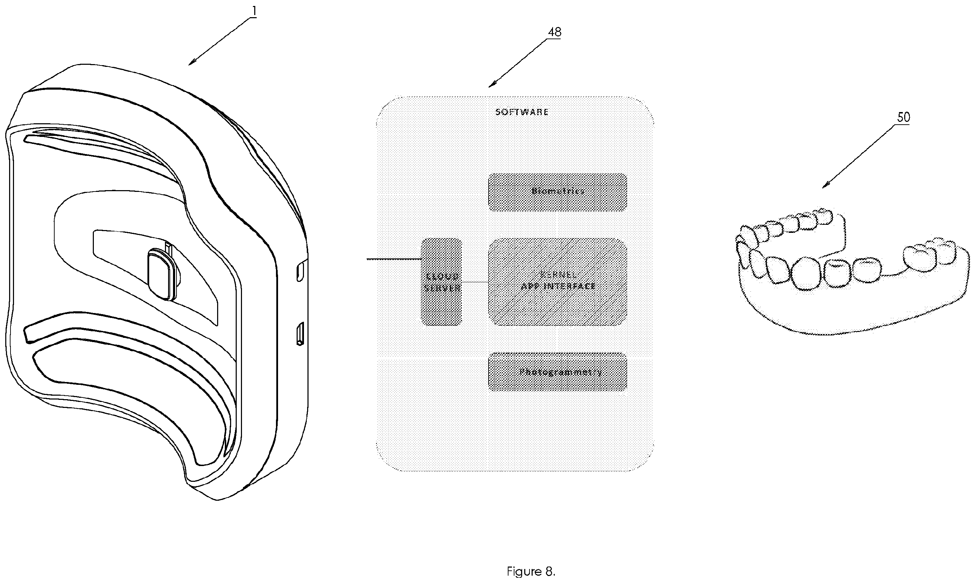

The captured digital images are stored temporarily in the device memory of the device 1 and are then transmitted to a cloud-based image processor 48 (see Figure 8) by the wireless communication device. The transmission time may depend on the speed of the wireless connection. The transmitted raw digital images are collected and organised into designated folders and stamped with the date and capture time, for example. The digital images taken by each digital camera are then processed by the image processor 48 to generate three-dimensional images 50 of the upper and lower sets of teeth and gums of the user. (Only the three-dimensional image of the lower set of teeth and gums is shown in Figure 8 for conciseness.) More particularly, the first set of digital images captured by the first digital camera are processed using photogrammetry software to generate a three-dimensional image of the lower set of teeth and gums of the user, and the second set of digital images captured by the second digital camera are processed using photogrammetry software to generate a three-dimensional image of the upper set of teeth and gums of the user. The processing may also use biometric data of the user, i.e., body measurements and/or calculations related to human characteristics.

The generated three-dimensional images 50 may be transmitted or downloaded to an electronic device, including a mobile device such as a phone or tablet.

The device 1 is turned off by pressing the on/off button 38.

Although the device described above uses movable first and second digital cameras, it will be understood that in an alternative arrangement, which does not form part of the claimed invention, the device may include a plurality of first digital cameras arranged at different fixed positions along a first virtual line and a plurality of second digital cameras arranged at different fixed positions along a second virtual line. The first and second virtual lines may correspond substantially to the first and second arcuate tracks or paths mentioned above. The first virtual line may be determined by the shape of a first support or other attachment means to which the first digital cameras are mounted. Similarly, the second virtual line may be determined by the shape of a second support or other attachment means to which the second digital cameras are mounted. **In** such an alternative device, the actuator for moving the first and second digital cameras (i.e., the Bowden mechanism) may be omitted.

## Claims

1. A portable, hand-held, dental imaging device (1) comprising:
an outer housing (2);
an electric or mechanical actuator (24a, 24b, 26);
a first arcuate track (6);
a first digital camera positioned at an upper part of the outer housing (2) and configured to capture a plurality of first digital images of one of the upper and lower sets of teeth and gums of a user when the device (1) is placed adjacent the mouth of the user, the first digital camera configured to be movable by the electric or mechanical actuator (24a, 24b, 26) along the first arcuate track (6), and to capture each first digital image from a different position along the first arcuate track (6);
a second arcuate track (16);
a second digital camera positioned at a lower part of the outer housing (2) and configured to capture a plurality of second digital images of the other one of the upper and lower sets of teeth and gums of a user when the device (1) is placed adjacent the mouth of the user, the second digital camera configured to be movable by the electric or mechanical actuator (24a, 24b, 26) or by a separate electric or mechanical actuator of the device (1) along the second arcuate track (16), and to capture each second digital image from a different position along the second arcuate track (16); and
a dental retractor (32), the dental retractor (32) being insertable into the mouth of the user in use to retract the cheeks and expose the teeth and gums of the user and to position the device (1) relative to and outside the mouth of the user when the plurality of first digital images are being captured by the first digital camera and the plurality of second digital images are being captured by the second digital camera.

2. A device (1) according to claim 1, further comprising a first lighting device (28) configured to illuminate the one of the upper and lower sets of teeth and gums of the user.

3. A device (1) according to any claim 1 or claim 2, further comprising a second lighting device (30) configured to illuminate the other one of the upper and lower sets of teeth and gums of the user.

4. A device (1) according to any preceding claim, wherein the dental retractor (32) is configured to be removable.

5. A device (1) according to any preceding claim, wherein the first digital camera is orientated downwards towards the lower set of teeth and gums of the user and the plurality of first digital images are digital images of the lower set of teeth and gums of the user, and wherein the second digital camera is orientated upwards towards the upper set of teeth and gums of the user and the plurality of second digital images are digital images of the upper set of teeth and gums of the user.

6. A device (1) according to any preceding claim, further comprising one or more of:
a processor and a memory device,
a wireless communication device,
a power source,
one or more control buttons (38, 40), and
a speaker device.

7. A system comprising the portable, hand-held, dental imaging device (1) according to any preceding claim and an image processor (48), wherein the image processor (48) is configured to process the plurality of first digital images captured by the dental imaging device (1) to generate a three-dimensional image (50) of the one of the upper and lower sets of teeth and gums of the user.

8. A system according to claim 7, wherein the image processor (48) is configured to process the plurality of second digital images captured by the dental imaging device (1) to generate a three-dimensional image (50) of the other one of the upper and lower sets of teeth and gums of the user.

9. A method of generating a three-dimensional image of the teeth and gums of a user using a portable, hand-held, dental imaging device (1) according to any of claims 1 to 6, the method comprising:
inserting the dental retractor (32) into the mouth of the user and placing the device (1) adjacent and outside the mouth of the user,
using the first digital camera to capture a plurality of first digital images of one of the upper and lower sets of teeth and gums of the user, each first digital image being captured from a different position along the first arcuate track (6), and
processing the plurality of first digital images to generate a three-dimensional image (50) of the one of the upper and lower sets of teeth and gums of the user.

10. A method according to claim 9, further comprising using the device (1) to illuminate the one of the upper and lower sets of teeth and gums of the user.

11. A method according to claim 9 or claim 10, wherein the plurality of first digital images are transmitted from the device (1) to an image processor (48) for generating the three-dimensional image (50) of the one of the upper and lower sets of teeth and gums of the user.

12. A method according to any of claims 9 to 11, wherein the method further comprises:
using the second digital camera to capture a plurality of second digital images of the other one of the upper and lower sets of teeth and gums of the user, each second digital image being captured from a different position along the second arcuate track (16), and
processing the plurality of second digital images to generate a three-dimensional image of the other one of the upper and lower sets of teeth and gums of the user.

13. A method according to claim 12, further comprising using the device (1) to illuminate the other one of the upper and lower sets of teeth and gums of the user.

14. A method according to claim 12 or claim 13, wherein the plurality of second digital images are transmitted from the device (1) to an image processor for generating the three-dimensional image of the other one of the upper and lower sets of teeth and gums of the user.

15. A method according to any of claims 12 to 14, wherein the first and second digital cameras are moved simultaneously along the respective first and second arcuate tracks (6, 16).

## Patentansprüche

1. Tragbare, handgehaltene dentale Bildgebungsvorrichtung (1), umfassend:
ein Außengehäuse (2);
einen elektrischen oder mechanischen Aktuator (24a, 24b, 26);
eine erste bogenförmige Führung (6);
eine erste Digitalkamera, die an einem oberen Teil des Außengehäuses (2) positioniert und so ausgebildet ist, dass sie eine Vielzahl von ersten digitalen Bildern einer der oberen und unteren Zahnreihen und des Zahnfleisches eines Benutzers aufnimmt, wenn die Vorrichtung (1) neben dem Mund des Benutzers platziert wird, wobei die erste Digitalkamera so ausgebildet ist, dass sie durch den elektrischen oder mechanischen Aktuator (24a, 24b, 26) entlang der ersten bogenförmigen Führung (6) bewegbar ist und jedes erste digitale Bild von einer anderen Position entlang der ersten bogenförmigen Führung (6) aufnimmt;
eine zweite bogenförmige Führung (16);
eine zweite Digitalkamera, die an einem unteren Teil des Außengehäuses (2) positioniert und so ausgebildet ist, dass sie eine Vielzahl von zweiten digitalen Bildern der anderen von den oberen und unteren Zahnreihen und des Zahnfleisches eines Benutzers aufnimmt, wenn die Vorrichtung (1) neben dem Mund des Benutzers platziert wird, wobei die zweite Digitalkamera so ausgebildet ist, dass sie durch den elektrischen oder mechanischen Aktuator (24a, 24b, 26) oder durch einen separaten elektrischen oder mechanischen Aktuator der Vorrichtung (1) entlang der zweiten bogenförmigen Führung (16) bewegbar ist und jedes zweite digitale Bild von einer anderen Position entlang der zweiten bogenförmigen Führung (16) aufnimmt; und
einen dentalen Retraktor (32), wobei der dentale Retraktor (32) bei der Verwendung in den Mund des Benutzers einführbar ist, um die Wangen zurückzuziehen und die Zähne und das Zahnfleisch des Benutzers freizulegen sowie die Vorrichtung (1) relativ zu dem und außerhalb des Mundes des Benutzers zu positionieren, wenn die Vielzahl von ersten digitalen Bildern von der ersten Digitalkamera aufgenommen wird und die Vielzahl von zweiten digitalen Bildern von der zweiten Digitalkamera aufgenommen werden.

2. Vorrichtung (1) nach Anspruch 1, weiter umfassend eine erste Beleuchtungsvorrichtung (28), die so ausgebildet ist, dass sie die eine von den oberen und unteren Zahnreihen und das Zahnfleisch des Benutzers beleuchtet.

3. Vorrichtung (1) nach einem von Anspruch 1 oder Anspruch 2, weiter umfassend eine zweite Beleuchtungsvorrichtung (30), die so ausgebildet ist, dass sie die andere von den oberen und unteren Zahnreihen und das Zahnfleisch des Benutzers beleuchtet.

4. Vorrichtung (1) nach einem vorstehenden Anspruch, wobei der dentale Retraktor (32) so ausgebildet ist, dass er abnehmbar ist.

5. Vorrichtung (1) nach einem vorstehenden Anspruch, wobei die erste Digitalkamera nach unten in Richtung der unteren Zahnreihe und des Zahnfleischs des Benutzers ausgerichtet ist und die Vielzahl erster digitaler Bilder digitale Bilder der unteren Zahnreihe und des Zahnfleischs des Benutzers sind, und wobei die zweite Digitalkamera nach oben in Richtung der oberen Zahnreihe und des Zahnfleischs des Benutzers ausgerichtet ist und die Vielzahl von zweiten digitalen Bildern digitale Bilder der oberen Zahnreihe und des Zahnfleischs des Benutzers sind.

6. Vorrichtung (1) nach einem vorstehenden Anspruch, weiter umfassend eines oder mehreres von:
einem Prozessor und einer Speichervorrichtung,
einer drahtlosen Kommunikationsvorrichtung,
einer Stromquelle,
einer oder mehreren Steuertasten (38, 40), und
einer Lautsprechervorrichtung.

7. System, das die tragbare, handgehaltene dentale Bildgebungsvorrichtung (1) nach einem vorstehenden Anspruch und einen Bildprozessor (48) umfasst, wobei der Bildprozessor (48) so ausgebildet ist, dass er die Vielzahl von ersten digitalen Bildern, die von der dentalen Bildgebungsvorrichtung (1) aufgenommen wurden, verarbeitet, um ein dreidimensionales Bild (50) der einen von den oberen und unteren Zahnreihen und des Zahnfleischs des Benutzers zu erzeugen.

8. System nach Anspruch 7, wobei der Bildprozessor (48) so ausgebildet ist, dass er die Vielzahl von zweiten digitalen Bildern, die von der dentalen Bildgebungsvorrichtung (1) aufgenommen wurden, verarbeitet, um ein dreidimensionales Bild (50) der anderen von den oberen und unteren Zahnreihen und des Zahnfleischs des Benutzers zu erzeugen.

9. Verfahren zum Erzeugen eines dreidimensionalen Bildes der Zähne und des Zahnfleischs eines Benutzers unter Verwendung einer tragbaren, handgehaltenen dentalen Bildgebungsvorrichtung (1) nach einem der Ansprüche 1 bis 6, wobei das Verfahren umfasst:
Einführen des dentalen Retraktors (32) in den Mund des Benutzers und Platzieren der Vorrichtung (1) neben und außerhalb des Mundes des Benutzers,
Verwenden der ersten Digitalkamera zum Aufnehmen einer Vielzahl von ersten digitalen Bildern einer der oberen und unteren Zahnreihen und des Zahnfleischs des Benutzers, wobei jedes erste digitale Bild von einer anderen Position entlang der ersten bogenförmigen Führung (6) aufgenommen wird, und
Verarbeiten der Vielzahl von ersten digitalen Bildern, um ein dreidimensionales Bild (50) der einen von den oberen und unteren Zahnreihen und des Zahnfleischs des Benutzers zu erzeugen.

10. Verfahren nach Anspruch 9, weiter umfassend das Verwenden der Vorrichtung (1) zum Beleuchten der einen von den oberen und unteren Zahnreihen und des Zahnfleischs des Benutzers.

11. Verfahren nach Anspruch 9 oder Anspruch 10, wobei die Vielzahl erster digitaler Bilder von der Vorrichtung (1) an einen Bildprozessor (48) übertragen wird, um das dreidimensionale Bild (50) der einen von den oberen und unteren Zahnreihen und des Zahnfleischs des Benutzers zu erzeugen.

12. Verfahren nach einem der Ansprüche 9 bis 11, wobei das Verfahren weiter umfasst:
Verwenden der zweiten Digitalkamera zum Aufnehmen einer Vielzahl von zweiten digitalen Bildern der anderen von den oberen und unteren Zahnreihen und des Zahnfleischs des Benutzers, wobei jedes zweite digitale Bild von einer anderen Position entlang der zweiten bogenförmigen Führung (16) aufgenommen wird, und
Verarbeiten der Vielzahl von zweiten digitalen Bildern, um ein dreidimensionales Bild der anderen von den oberen und unteren Zahnreihen und dem Zahnfleisch des Benutzers zu erzeugen.

13. Verfahren nach Anspruch 12, weiter umfassend das Verwenden der Vorrichtung (1) zum Beleuchten der anderen von den oberen und unteren Zahnreihen und des Zahnfleischs des Benutzers.

14. Verfahren nach Anspruch 12 oder Anspruch 13, wobei die Vielzahl von zweiten digitalen Bildern von der Vorrichtung (1) an einen Bildprozessor übertragen wird, um das dreidimensionale Bild der anderen von den oberen und unteren Zahnreihen und des Zahnfleischs des Benutzers zu erzeugen.

15. Verfahren nach einem der Ansprüche 12 bis 14, wobei die erste und die zweite Digitalkamera gleichzeitig entlang der jeweiligen ersten und zweiten bogenförmigen Führungen (6, 16) bewegt werden.

## Revendications

1. Dispositif (1) d'imagerie dentaire portable, tenu à la main, comprenant :
un boîtier externe (2) ;
un actionneur électrique ou mécanique (24a, 24b, 26) ;
un premier rail arqué (6) ;
une première caméra numérique, positionnée au niveau d'une partie supérieure du boîtier externe (2) et configurée pour capturer une pluralité de premières images numériques de l'un parmi l'ensemble supérieur de dents et de gencives et l'ensemble inférieur de dents et de gencives d'un utilisateur lorsque le dispositif (1) est placé de manière adjacente à la bouche de l'utilisateur, la première caméra numérique étant configurée pour être déplacée par l'actionneur électrique ou mécanique (24a, 24b, 26) le long du premier rail arqué (6), et pour capturer chaque première image numérique depuis une position différente le long du premier rail arqué (6) ;
un deuxième rail arqué (16) ;
une deuxième caméra numérique, positionnée au niveau d'une partie inférieure du boîtier externe (2) et configurée pour capturer une pluralité de deuxièmes images numériques de l'autre parmi l'ensemble supérieur de dents et de gencives et l'ensemble inférieur de dents et de gencives d'un utilisateur lorsque le dispositif (1) est placé de manière adjacente à la bouche de l'utilisateur, la deuxième caméra numérique étant configurée pour être déplacée par l'actionneur électrique ou mécanique (24a, 24b, 26) ou par un actionneur électrique ou mécanique séparé du dispositif (1) le long du deuxième rail arqué (16), et pour capturer chaque deuxième image numérique depuis une position différente le long du deuxième rail arqué (16) ; et
un écarteur dentaire (32), l'écarteur dentaire (32) étant insérable dans la bouche de l'utilisateur lors de l'utilisation pour rétracter les joues et exposer les dents et les gencives de l'utilisateur et pour positionner le dispositif (1) par rapport à la bouche de l'utilisateur et à l'extérieur de celle-ci lorsque la pluralité de premières images numériques est capturée par la première caméra numérique et la pluralité de deuxièmes images numériques est capturée par la deuxième caméra numérique.

2. Dispositif (1) selon la revendication 1, comprenant en outre un premier dispositif d'éclairage (28) configuré de manière à éclairer l'un parmi l'ensemble supérieur de dents et de gencives et l'ensemble inférieur de dents et de gencives de l'utilisateur.

3. Dispositif (1) selon la revendication 1 ou la revendication 2, comprenant en outre un deuxième dispositif d'éclairage (30) configuré de manière à éclairer l'autre parmi l'ensemble supérieur de dents et de gencives et l'ensemble inférieur de dents et de gencives de l'utilisateur.

4. Dispositif (1) selon une quelconque revendication précédente, dans lequel l'écarteur dentaire (32) est configuré de manière à être amovible.

5. Dispositif (1) selon une quelconque revendication précédente, dans lequel la première caméra numérique est orientée vers le bas en direction de l'ensemble inférieur de dents et de gencives de l'utilisateur et la pluralité de premières images numériques est constituée d'images numériques de l'ensemble inférieur de dents et de gencives de l'utilisateur, et dans lequel la deuxième caméra numérique est orientée vers le haut en direction de l'ensemble supérieur de dents et de gencives de l'utilisateur et la pluralité de deuxièmes images numériques est constituée d'images numériques de l'ensemble supérieur de dents et de gencives de l'utilisateur.

6. Dispositif (1) selon une quelconque revendication précédente, comprenant en outre un ou plusieurs parmi :
un processeur et un dispositif de mémoire,
un dispositif de communication sans fil,
une source de puissance,
un ou plusieurs boutons de commande (38, 40), et
un haut-parleur.

7. Système comprenant le dispositif (1) d'imagerie dentaire portable, tenu à la main, selon une quelconque revendication précédente et un processeur d'images (48), dans lequel le processeur d'images (48) est configuré de manière à traiter la pluralité de premières images numériques capturées par le dispositif d'imagerie dentaire (1) pour générer une image tridimensionnelle (50) de l'un parmi l'ensemble supérieur de dents et de gencives et l'ensemble inférieur de dents et de gencives de l'utilisateur.

8. Système selon la revendication 7, dans lequel le processeur d'images (48) est configuré de manière à traiter la pluralité de deuxièmes images numériques capturées par le dispositif (1) d'imagerie dentaire afin de générer une image tridimensionnelle (50) de l'autre parmi l'ensemble supérieur de dents et de gencives et l'ensemble inférieur de dents et de gencives de l'utilisateur.

9. Procédé de génération d'une image tridimensionnelle des dents et des gencives d'un utilisateur en utilisant un dispositif (1) d'imagerie dentaire portable, tenu à la main, selon l'une quelconque des revendications 1 à 6, le procédé comprenant :
le fait d'insérer l'écarteur dentaire (32) dans la bouche de l'utilisateur et de placer le dispositif (1) de manière adjacente à la bouche de l'utilisateur et à l'extérieur de celle-ci,
le fait d'utiliser la première caméra numérique pour capturer une pluralité de premières images numériques de l'un parmi l'ensemble supérieur de dents et de gencives et l'ensemble inférieur de dents et de gencives de l'utilisateur, chaque première image numérique étant capturée depuis une position différente le long du premier rail arqué (6), et
le fait de traiter la pluralité de premières images numériques afin de générer une image tridimensionnelle (50) de l'un parmi l'ensemble supérieur de dents et de gencives et l'ensemble inférieur de dents et de gencives de l'utilisateur.

10. Procédé selon la revendication 9, comprenant en outre le fait d'utiliser le dispositif (1) de manière à éclairer l'un parmi l'ensemble supérieur de dents et de gencives et l'ensemble inférieur de dents et de gencives de l'utilisateur.

11. Procédé selon la revendication 9 ou la revendication 10, dans lequel la pluralité de premières images numériques est transmise du dispositif (1) à un processeur d'images (48) afin de générer l'image tridimensionnelle (50) de l'un parmi l'ensemble supérieur de dents et de gencives et l'ensemble inférieur de dents et de gencives de l'utilisateur.

12. Procédé selon l'une quelconque des revendications 9 à 11, dans le quel le procédé comprend en outre :
le fait d'utiliser la deuxième caméra numérique pour capturer une pluralité de deuxièmes images numériques de l'autre parmi l'ensemble supérieur de dents et de gencives et l'ensemble inférieur de dents et de gencives de l'utilisateur, chaque deuxième image numérique étant capturée depuis une position différente le long du deuxième rail arqué (16), et
le fait de traiter la pluralité de deuxièmes images numériques de manière à générer une image tridimensionnelle de l'autre parmi l'ensemble supérieur de dents et de gencives et l'ensemble inférieur de dents et de gencives de l'utilisateur.

13. Procédé selon la revendication 12, comprenant en outre de fait d'utiliser le dispositif (1) de manière à éclairer l'autre parmi l'ensemble supérieur de dents et de gencives et l'ensemble inférieur de dents et de gencives de l'utilisateur.

14. Procédé selon la revendication 12 ou la revendication 13, dans lequel la pluralité de deuxièmes images numériques est transmise du dispositif (1) à un processeur d'images pour générer l'image tridimensionnelle de l'autre parmi l'ensemble supérieur de dents et de gencives et l'ensemble inférieur de dents et de gencives de l'utilisateur.

15. Procédé selon l'une quelconque des revendications 12 à 14, dans lequel les première et deuxième caméras numériques sont déplacées simultanément le long des premier et deuxième rails arqués respectifs (6, 16).
